# DEMANDE DE BREVET EUROPEEN

(11) **EP 1 897 592 A1**
(43) Date de publication de la demande: **12.03.2008**
(21) Numéro de dépôt: 07111439.1
(22) Date de dépôt: 29.06.2007
(51) Int. Cl.: A61Q 13/00, A61K 8/35, A61K 8/368, A61K 8/40

(54) **Composition parfumée colorée comprenant l'association d'un composé beta,beta '-diphénylacrylate d'alkyle ou alpha -cyano-beta,beta '-diphénylacrylate d'alkyle et d'au moins un filtre organique uv-a soluble**

(30) Priorité: 30.08.2006 FR 0607619
(71) Demandeur: L'ORÉAL, 75008 Paris (FR)
(72) Inventeur: Picard-Lesboueyries, Elisabeth, 78140 Velizy (FR); Lecomte Desgranges, Liliane, 94320 Thiais (FR)
(74) Mandataire: Miszputen, Laurent

(57) **Abrégé**

L'invention se rapporte à une composition parfumée colorée comprenant dans un milieu cosmétiquement acceptable
a) au moins 1% en poids d'une substance parfumante ;
b) au moins un alcool volatil et/ou une huile de silicone volatile
c) au moins un colorant soluble dans ledit milieu ;
d) au moins 0,2% en poids d'au moins un composé β,β'-diphénylacrylate d'alkyle ou α-cyano-β,β'-diphénylacrylate d'alkyle et
e) au moins un filtre organique UV-A soluble dans ledit milieu.
f) et éventuellement de l'eau.

L'invention se rapporte également à l'utilisation de l'association d'au moins 0,2% en poids d'un composé β,β'-diphénylacrylate d'alkyle ou α-cyano-β,β'-diphénylacrylate d'alkyle et d'au moins un filtre organique UV-A soluble définie ci-dessus dans une composition parfumée colorée comprenant dans un milieu cosmétiquement acceptable
e) au moins 1% en poids d'au moins une substance parfumante ;
f) au moins un alcool volatil et/ou une huile de silicone volatile
g) au moins un colorant soluble dans ledit milieu,
h) et éventuellement de l'eau ,
comme agent stabilisant de la couleur de ladite composition.

L'invention se rapporte également à un procédé cosmétique de parfumage des matières kératiniques humaines et notamment de la peau, des lèvres et des phanères, comprenant l'application sur les matières kératiniques de la composition définie précedemment.

## Description

L'invention se rapporte à une composition parfumée colorée comprenant dans un milieu cosmétiquement acceptable :
a) au moins 1% en poids par rapport au poids total de la composition d'au moins une substance parfumante ;
b) au moins un alcool volatil et/ou une huile de silicone volatile,
c) au moins un colorant soluble dans ledit milieu,
d) au moins 0,2% en poids par rapport au poids total de la composition d'au moins un composé β,β'-diphénylacrylate d'alkyle ou α-cyano-β,β'-diphénylacrylate d'alkyle par rapport au poids total de la composition et
e) au moins un filtre organique UV-A soluble dans ledit milieu.
f) et éventuellement de l'eau.

On sait qu'un parfum est l'association de différentes substances odorantes qui s'évaporent à des périodes différentes. Chaque parfum présente ce que l'on appelle une « note de tête » qui est l'odeur diffusant en premier lors de l'application du parfum ou lors de l'ouverture du récipient le contenant, une « note de coeur ou corps » qui correspond au parfum complet (émission pendant quelques heures après la « note de tête ») et une « note de fond » qui est l'odeur la plus persistante (émission pendant plusieurs heures après la « note de coeur »). La persistance de la note de fond correspond à la rémanence du parfum.

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

II est courant d'incorporer du parfum dans un certain nombre de produits ou compositions, en particulier cosmétiques et dermatologiques telles que des eaux fraîches, eaux de toilette, des eaux de parfum, des lotions après rasage, des eaux de soin. Pour des raisons esthétiques et de coût de fabrication, on colore le jus des parfums en ajoutant une quantité efficace de colorant soluble dans le support de la formulation (généralement alcoolique ou hydroalcoolique) plutôt que de teindre ou laquer le flacon qui est une opération industrielle plus onéreuse. La couleur mise au point dans ces formulations parfumées doit rester stable aussi bien dans le temps, qu'exposée à la lumière. On ajoute généralement un système filtrant et/ou un système antioxydant.

On connait par exemple dans la demande WO2005/042828 des compositions parfumantes colorées comprenant comme système stabilisant un dérivé de pipéridinol (ie Tris(tétraméthylhydroxypipéridinol) citrate - Tinoguard Q) associé à un filtre UV organique choisi parmi lesdérivés de dibenzoylméthane, les cinnamates, les dérivés de camphres et le s-triazines. L'exemple 6 décrit notamment une eau de toilette comprenant un parfum, un colorant, 0,1% d'un mélange de Butylmethoxydibenzoylmethane et d'octocrylene, un stabilsant du type pipéridinol

On connaît également dans la demande WO00/25370 des eaux de toilettes colorées stabilisées par un composé benzotriale particulier et/ou une triazine particulière comme par exemple le Sodium Benzotriazolyl Butylphenol Sulfonate comme le produit vendu sous le nom « TINOGUARD HS » par la société CIBA-GEIGY ; le Benzotriazolyl dodécyl p-Cresol comme le produit vendu sous le nom « TINOGUARD TL » par la société CIBA-GEIGY. comme le produit vendu sous le nom commercial « CIBAFAST H LIQUID » par la société CIBA-GEIGY. Bumetrizole comme le produit vendu sous le nom « TINOGUARD AS » par la société CIBA-GEIGY.

Cependant, certains de ces systèmes filtrants et/ou antioxydants sont inefficaces et/ou ont tendance à dénaturer la couleur recherchée.

II subsiste le besoin de produits parfumants colorés ne présentant pas les inconvénients des produits de l'art antérieur, et notamment le besoin de produits parfumants colorés dont la couleur reste stable dans le temps et sous les effets de la lumière.

La demanderesse a découvert de manière surprenante que cet objectif pouvait être atteint en utilisant l'association d'au moins un composé β,β'-diphénylacrylate d'alkyle ou α-cyano-β,β'-diphénylacrylate d'alkyle et d'au moins un filtre organique UV-A soluble dans ledit milieu.

Cette découverte est à la base de l'invention.

L'invention se rapporte à une composition parfumée colorée comprenant dans un milieu cosmétiquement acceptable
a) au moins 1% en poids par rapport au poids total de la composition d'au moins une substance parfumante ;
b) au moins un alcool volatil et/ou une huile de silicone volatile,
c) au moins un colorant soluble dans ledit milieu,
d) au moins 0,2% en poids par rapport au poids total de la composition d'au moins un composé β,β'-diphénylacrylate d'alkyle ou α-cyano-β,β'-diphénylacrylate d'alkyle par rapport au poids total de la composition et
e) au moins un filtre organique UV-A soluble dans ledit milieu.
f) et éventuellement de l'eau.

L'invention se rapporte également à l'utilisation d'au moins 0,2% en poids d'au moins un composé β,β'-diphénylacrylate d'alkyle ou α-cyano-β,β'-diphénylacrylate d'alkyle et d'au moins un filtre organique UVA soluble définie ci-dessus dans une composition parfumée colorée comprenant dans un milieu cosmétiquement acceptable :
a) au moins 1% en poids par rapport au poids total de la composition d'au moins une substance parfumante ;
b) au moins un alcool volatil et/ou une huile de silicone volatile
c) au moins un colorant soluble dans ledit milieu,
d) et éventuellement de l'eau,
comme agent stabilisant de la couleur de ladite composition.

L'invention a encore pour objet un procédé cosmétique de parfumage des matières kératiniques humaines et notamment de la peau, des lèvres et des phanères, comprenant l'application sur les matières kératiniques de la composition telle que définie ci-dessus.

Par « substance parfumante», on entend tout parfum ou arôme susceptible de dégager une odeur agréable.

De préférence, la quantité de substance(s) parfumante(s) varie de 1 à 30 % en poids, mieux de 2 à 25% en poids par rapport au poids total de la composition.

On entend par milieu cosmétiquement acceptable dans la composition de l'invention, un milieu non toxique et susceptible d'être appliqué sur la peau (y compris l'intérieur des paupières), les lèvres, les ongles ou les cheveux d'êtres humains.

Par « filtre organique UV-A », on entend tout composé comprenant au moins un groupe chromophore susceptible de filtrer les radiations UV dans le domaine des radiations UV-A à savoir de longueur d'onde comprise entre 320 et 400 nm.

Par « soluble dans le milieu », on entend tout composé susceptible d'être complètement dissous dans une phase comprenant au moins un alcool volatil et/ou au moins une huile siliconée volatile et éventuellement en plus de l'eau.

Par « alcool volatil », tout composé comprenant au moins une fonction alcool ayant une pression de vapeur à 20 °C supérieure à 17,5 mm de mercure.

Par « huile », on entend, au sens de l'invention, un corps gras, non soluble dans l'eau, et liquide à température ambiante et pression atmosphérique.

Par « huile de silicone volatile », on entend, au sens de l'invention, tout composé siliconé susceptible de s'évaporer au contact de la peau ou de la fibre kératinique en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de1,3 Pa à 1300 Pa (0,01 à 10 mm de Hg).

Comme substance parfumante, on peut utiliser dans la composition de l'invention, les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance parfumante d'origine synthétique, on peut citer par exemple les composés du type ester, éther, aldéhyde, cétone, alcool aromatique et hydrocarbure.

Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.

Comme éthers, on peut citer le benzyléthyléther.

Comme aldéhydes, on peut citer par exemple les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal.

Comme cétones, on peut citer par exemple les ionones comme l'alpha-isométhylionone, et la méthylcédrylcétone.

Parmi les alcool aromatiques et notamment terpéniques, on peut citer l'anéthol, le citronellol, l'eugénol, l'isoeugénol, le géraniol, le linalol, le phényléthylalcool et le terpinéol.

Comme hydrocarbures, on peut citer notamment les terpènes. Ces composés se présentent souvent sous forme de mélange de deux ou plus de ces substances odorantes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

On utilise de préférence comme substance parfumante, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

Selon un mode préféré de réalisation de l'invention, on utilise un mélange de différentes substances parfumante qui engendrent en commun une note plaisante pour l'utilisateur. Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges

Les β,β'-diphénylacrylates d'alkyle et α-cyano-β,β'-diphénylacrylates d'alkyle utilisables selon la présente invention sont choisis parmi ceux répondant à la formule (I) suivante : 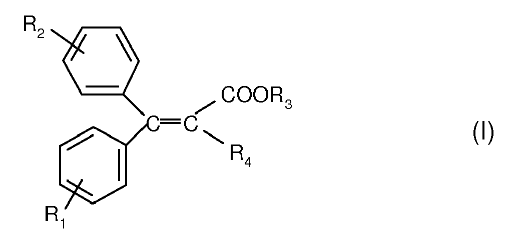 dans laquelle :
- R₁ et R₂, identiques ou différents, sont en position méta ou para et sont choisis parmi : l'hydrogène; un radical alcoxy en C₁-C₈ à chaîne droite ou ramifiée; un radical alkyle en C₁-C₄ à chaîne droite ou ramifiée;
- R₃ représente un radical alkyle en C₁-C₁₂ à chaîne droite ou ramifiée;
- R₄ représente un atome d'hydrogène ou un radical -CN.

Parmi les β,β'-diphénylacrylates d'alkyle et α-cyano-β,β'-diphénylacrylates d'alkyle utilisables selon la présente invention, on préfère plus particulièrement l'α-cyano-β,β' diphénylacrylate de 2-éthylhexyle ou encore l'α-cyano-β,β'-diphénylacrylate d'éthyle.

L'α-cyano-β,β' diphénylacrylate de 2-éthylhexyle, encore appelé octocrylène, est connu pour être un filtre lipophile absorbant dans les UVB. Il est disponible commercialement et vendu notamment sous la dénomination de "UVINUL N 539" par la Société BASF. II répond à la formule suivante : 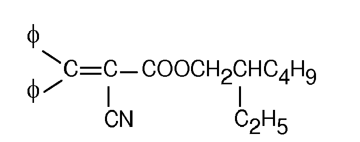 dans laquelle φ désigne un radical phényle.

L'α-cyano-β,β' diphénylacrylate d'éthyle, encore appelé étocrylène, est également un filtre liposoluble absorbant dans les UVB. II est disponible commercialement et vendu notamment sous la dénomination de "UVINUL N 35" par la Société BASF. II répond à la formule suivante : 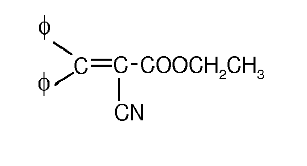 dans laquelle φ désigne un radical phényle.

L'α-cyano-β,β' diphénylacrylate de 2-éthylhexyle est plus particulièrement préféré.

De préférence, le β,β'-diphénylacrylate d'alkyle ou α-cyano-β,β'-diphénylacrylate d'alkyle est présent dans les compositions selon l'invention à une teneur d'au moins 0,2% et de préférence de 0,5 à 3% en poids, et plus particulièrement de 0,5 à 1,5% en poids par rapport au poids total de la composition.

Ainsi, lorsqu'on ajoute en quantité suffisante de l'association selon l'invention à une composition parfumée colorée de l'invention on observe une augmentation de la stabilité de la couleur dans le temps et sous les effets de la lumière.

Les filtres UV-A solubles dans le milieu de la composition, peuvent être de préférence choisis parmi les dérivés de benzophénone, les dérivés de dibenzoylméthane, les dérivés de benzylidène camphre sulfoniques tels que ceux décrits dans le brevet FR2528420, les silicones benzotriazoles tels que ceux cités dans le brevet EP660701.

Parmi les dérivés de benzophénones solubles dans le milieu de la composition, on peut citer :
Benzophenone-2 vendu sous le nom commercial « UVINUL D50 » par BASF
Benzophenone-3 ou Oxybenzone, vendu sous le nom commercial « UVINUL M40 » par BASF,
2-(4-diethylamino-2-hydroxybenzoyl)-benzoate de n-hexyle vendu sous le nom commercial« UVINUL A+» par BASF ou « UVINUL A +B » sous forme de mélange avec l'octylmethoxycinnamate.

Parmi les dérivés de benzylidène camphre sulfoniques solubles dans le milieu de la composition, on peut citer :
Terephthalylidene Dicamphor Sulfonic Acid fabriqué sous le nom « MEXORYL SX » par CHIMEX,

Parmi les silicones benzotriazoles solubles dans le milieu de la composition, on citera plus particulièrement les composés de formule générale (II) suivante : 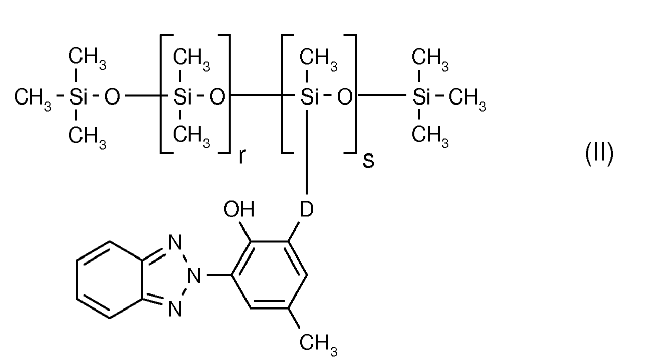 avec
0 ≤ r ≤ 15 , de préférence 0 ≤ r ≤ 10
1 ≤ s ≤ 5 , de préférence 1 ≤ s ≤ 3
et où D représente le radical divalent : 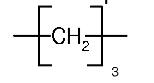 ou 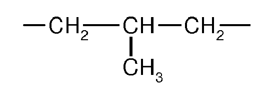 et plus particulièrement le composé Drometrizole Trisiloxane de formule (II) avec r = 0 et s = 1 et 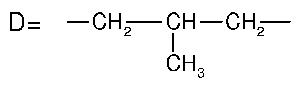 vendu sous le nom « Silatrizole » par RHODIA CHIMIE.

Parmi les dérivés du dibenzoylméthane conformes à l'invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dérivés du dibenzoylméthane mentionnés ci-dessus, on utilisera en particulier le 4-isopropyl-dibenzoylméthane, vendu sous la dénomination de "EUSOLEX 8020" par la Société MERCK, et répondant à la formule suivante :

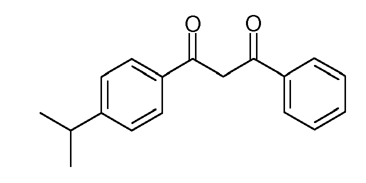

On préfère tout particulièrement mettre en oeuvre le 4-(ter.-butyl) 4'-méthoxy dibenzoylméthane ou Butyl Methoxy Dibenzoylmethane, proposé à la vente sous la dénomination commerciale de "PARSOL 1789" par la Société DSM ; ce filtre répond à la formule suivante :

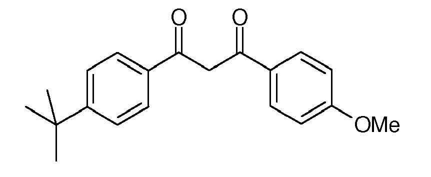

Parmi les filtres UV-A solubles conformes à l'invention, on choisira plus particulièrement ceux du type dibenzoylméthane et encore plus particulièrement le 4-(ter.-butyl) 4'-méthoxy dibenzoylméthane ou Butyl Methoxy Dibenzoylmethane.

Le ou les filtres UV-A solubles conformes à l'invention sont de préférence présents dans les compositions selon l'invention à une teneur allant de 0,01% à 3% en poids, et plus particulièrement de 0,5 à 1,5% en poids par rapport au poids total de la composition.

Le milieu cosmétiquement acceptable conforme à la présente invention contient au moins un alcool volatil et/ou une huile de silicone volatile et éventuellement de l'eau. De façon préférentielle le milieu de la composition contient de l'eau dans une quantité allant de préférence de 0,01 % à 50 % et plus préférentiellement, de 0,5 % à 25 % en poids par rapport au poids total de la composition.

Les alcools volatils conformes à la présente invention sont choisis de préférence parmi monoalcools inférieurs en C₁-C₅ peuvent être choisis parmi le méthanol, l'éthanol, propanol, l'isopropanol, le n-butanol, l'isobutanol le t-butanol et plus particulièrement l'éthanol.

Le ou les alcools volatils sont présents de préférence dans des quantités allant de 40 à 80% et plus préférentiellement dans des quantités allant de 55 à 80% en poids par rapport au poids total de la composition.

Comme huiles siliconées volatiles, on peut citer par exemple les huiles de silicones linéaires ou cycliques volatiles, notamment celles ayant une viscosité ≤ 6 centistokes (6.10⁻⁶ m²/s), et ayant notamment de 2 à 10 atomes de silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile ou les huiles siliconées volatiles sont présentes de préférence de 40 à 98,5% de préférence dans des concentrations allant de 10% à 80 % en poids par rapport au poids total de la composition.

Selon une forme de l'invention, on utilisera en plus un ou plusieurs filtres UV organiques supplémentaires actifs dans l'UV-B solubles dans le milieu de la composition.

Les filtres UV organiques sont notamment choisis parmi les dérivés cinnamiques ; les dérivés salicyliques, les dérivés du camphre cationiques alkylsulfates tels que ceux décrits dans le brevet FR2199971 et leurs mélanges.

Comme exemples d'agents photoprotecteurs organiques supplémentaires, on peut citer ceux désignés ci-dessous sous leur nom INCI :

### Dérivés cinnamiques :

Ethylhexyl Methoxycinnamate vendu notamment sous le nom commercial PARSOL MCX par HOFFMANN LAROCHE,

### Dérivés salicyliques :

Homosalate vendu sous le nom « Eusolex HMS » par Rona/EM Industries, Ethylhexyl Salicylate vendu sous le nom « NEO HELIOPAN OS » par Haarmann et REIMER,

### Dérivés du camphre cationiques alkylsulfates :

Camphor Benzalkonium Methosulfate fabriqué sous le nom « MEXORYL SO » par CHIMEX,
et leurs mélanges.

Les agents photoprotecteurs supplémentaires sont présents de préférence dans les compositions dans des quantités allant de 0,01 à 3 % en poids et plus préférentiellement de 0,5 à 1,5% en poids par rapport au poids total de la composition.

Les colorants solubles conformes à l'invention sont en de préférence des colorants hydrosolubles ou hydrophiles parmi lesquels, on peut citer :
le caramel, Yellow 5 , Acid Blue 9/ Blue 1, Green 5, Green 3 / Fast Green FCF 3, Orange 4, Red 4 / Food Red 1, Yellow 6, Acid Red 33 / Food Red 12, Red 40, le carmine de cochenille (CI 15850, CI 75470), Ext. Violet 2, Red 6-7, Ferric Ferrocyanide, Ultramarines, Acide Yellow 3 / Yellow 10, Acid Blue 3, Yellow 10.

Le ou les colorant (s) soluble (s) conformes à l'invention sont de préférence présents dans des quantités allant de 10⁻⁵ à 1 % du poids total de la composition, de préférence de 10⁻⁴ à 0,1% du poids total de la composition.

La composition de l'invention peut comprendre, en outre, tout additif usuellement utilisé dans le domaine des parfums choisi notamment parmi les antioxydants, les actifs cosmétiques ou dermatologiques comme par exemple les émollients ou adoucissants comme les huiles d'amande douce, de noyau d'abricot, les agents hydratants comme la glycérine, les agents apaisants comme l'a-bisabolol, l'allantoïne, aloes vera ; les vitamines, les acides gras essentiels, les agents répulsifs contre les insectes, les propulseurs, les peptisants, des charges, des nacres, des paillettes et leurs mélanges. Quand ils sont présents dans la composition de l'invention, ces additifs peuvent être présents en une quantité allant de 0,001 à 10 % et mieux de 0,01 à 5% en poids par rapport au poids total de la composition.

Parmi les antioxydants, on peut citer par exemple le BHA (tert-butyl-4-hydroxyanisole), le BHT (2,6-di-tert-butyl-p-crésol), les tocophérols comme la vitamine E et ses dérivés tels que l'acétate de tocophéryle.

Selon une forme particulière de l'invention, la composition parfumante colorée ne contiendra pas d'autre stabilisant de la couleur que l'association composé β,β'-diphénylacrylate d'alkyle ou α-cyano-β,β'-diphénylacrylate/filtre UVA soluble. Comme exemple de stabilisant de la couleur de parfums, on citera le Tris(tétraméthylhydroxypipéridinol) citrate tel que produit vendu sous le nom « TINOGUARD Q » par la société CIBA-GEIGY, le Sodium Benzotriazolyl Butylphenol Sulfonate comme le produit vendu sous le nom « TINOGUARD HS » par la société CIBA-GEIGY ; le Benzotriazolyl dodécyl p-Cresol comme le produit vendu sous le nom « TINOGUARD TL » par la société CIBA-GEIGY. comme le produit vendu sous le nom commercial « CIBAFAST H LIQUID » par la société CIBA-GEIGY. Bumetrizole comme le produit vendu sous le nom « TINOGUARD AS » par la société CIBA-GEIGY.

Bien entendu l'homme du métier veillera à choisir les éventuels additifs complémentaires et/ou leur quantité de telle manière que les propriétés avantageuses de la composition selon l'invention ne soient pas ou substantiellement pas altérées par l'adjonction envisagée.

L'invention s'applique non seulement aux produits parfumants colorés mais aussi aux produits de soin, de traitement de la peau, y compris du cuir chevelu, et des lèvres, contenant une substance odorante. La composition selon l'invention peut ainsi constituer une composition de parfumage, de soin, de traitement des matières kératiniques, et notamment se présenter sous forme d'eau fraîche, d'eau de toilette, d'eau de parfum, de lotion après-rasage, d'eau de soin, d'huile de soin siliconée ou hydrosiliconée. Elle peut également se présenter sous la forme d'une lotion biphasique parfumée (phase eau de toilette/phase huile hydrocarbonée et/ou huile siliconée).

L'invention a encore pour objet un procédé cosmétique de parfumage des matières kératiniques des êtres humains et notamment de la peau, des lèvres et des phanères, comprenant l'application sur les matières kératiniques de la composition telle que définie ci-dessus.

La composition selon l'invention peut être fabriquée par les procédés connus, généralement utilisés dans le domaine des lotions parfumées et colorées.

Les compositions selon l'invention sous forme de lotions peuvent être conditionnées sous forme de flacons. Elles peuvent également être appliquées sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane.

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations parfumantes suivantes ; les quantités sont indiquées en pourcentages en poids :

### Exemples

On prépare les compositions suivantes :

| Ingredients | Ex 1 (invention) | Ex 2 | Ex 3 | Ex 4 | Ex 5 |
|---|---|---|---|---|---|
| Parfum du type oriental (*) | 6,99 | 6,99 | 6,99 | 6,99 | 6,99 |
| BHT | 0,025 | 0,025 | 0,025 | 0,025 | 0,025 |
| Yellow 5 | 0.00094 | 0.00094 | 0.00094 | 0.00094 | 0.00094 |
| Green 5 | 0.00016 | 0.00016 | 0.00016 | 0.00016 | 0.00016 |
| Red 4 | 0,00041 | 0,00041 | 0,00041 | 0,00041 | 0,00041 |
| Octocrylene | 0,5 | - | - | - | 0,5 |
| Butyl Methoxy Dibenzoylmethane | 0,5 | 0,16 | - | - | - |
| Ethylhexyl salicylate | - | 0,15 | - | - | - |
| Octylmethoxycinnamate | - | 0,69 | - | 0,20 | - |
| 2-(4-diethylamino-2- | - | - | - | 0,10 | - |
| hydroxybenzoyl)-benzoate de n- | | | | | |
| hexyle | | | | | |
| Tris(tétraméthylhydroxypipéridinol) | - | - | 0,03 | - | - |
| citrate (Tinoguard Q) | | | | | |
| Benzophenone-3 | | 0,2 | 0,2 | - | |
| Ethanol | 71,25 | 71,25 | 71,25 | 71,25 | 71,25 |
| Eau | qsp 100 | qsp 100 | qsp 100 | qsp 100 | qsp 100 |

| | | | | | |
|---|---|---|---|---|---|
| (*) Parfum de la famille des orientaux caractérisé par: - ses notes de tête de type grenade, carambole, amaretto - ses notes de coeur de type jasmin, pivoine hibiscus rouge - ses notes de fond de type fève tonka, myrrhe, baume du Pérou | | | | | |

| Ingredients | Ex 6 (invention) | Ex 7 | Ex 8 |
|---|---|---|---|
| Parfum du type fruité floral (**) | 15,00 | 15,00 | 15,00 |
| BHT | 0,0059 | 0,0059 | 0,0059 |
| Red 4 | 0,000091 | 0,000091 | 0,000091 |
| Octocrylene | 0,58 | - | 0,58 |
| Butyl Methoxy Dibenzoylmethane | 0,58 | 0,58 | - |
| Ethanol | 71,36 | 71,36 | 71,36 |
| Eau | qsp 100 | qsp 100 | qsp 100 |

| | | | |
|---|---|---|---|
| (**) Parfum de la famille des fruité-floraux caractérisé par : - ses notes de tête de type poire et essence de poivre rose - ses notes de coeur de type rose otto, violette - ses notes de fond de type héliotrope et santal. | | | |

Chaque formule est conditionnée dans un flacon standard en verre de 50 ml.

Chaque flacon est déposé dans un simulateur solaire non réfrigéré pendant une durée de 16 heures selon le protocole suivant :

### Principe

Le principe de ces essais consiste à exposer les produits à tester à une source lumineuse irradiante dont la répartition spectrale est parfaitement définie et l'énergie émise parfaitement quantifiée.

Les sources lumineuses couramment utilisées sont des lampes au xénon émettant au travers :
- un filtre en quartz platiné qui détourne le rayonnement infra rouge et l'élimine vers le haut de l'appareil
- et un filtre en verre, qui en absorbant le rayonnement ultra violet court permet de simuler le rayonnement reçu derrière une vitrine d'exposition.

### Matériel

On utilise un appareil CPS Sun -test :
- L'éclairement fourni par une lampe au xénon entre 300 et 800nm est fixé à 765W/m2 (valeur réglée par le constructeur)
- La filtration optique est assurée par d'un filtre quartz avec revêtement IR et un verre à vitres spécial)

On observe ensuite la couleur de chaque flacon avant et après exposition à la lumière, à l'oeil nu et à l'aide d'un spectrophotomètre MINOLTA CM3600-d. On quantifie la couleur obtenue. Les résultats sous forme de moyennes sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la couleur de la composition.

### Résultats

| Compositions | L * | | a * | | b * | | Couleur observée à l'oeil nu avant et après exposition | |
|---|---|---|---|---|---|---|---|---|
| | avant et après exposition | | avant et après exposition | | avant et après exposition | | | |
| Ex 1 (invention) | 91,96 | 94,15 | -0,02 | -3,20 | 37,41 | 33,10 | Orangé | Orangé |
| Ex 2 | 89,95 | 93,95 | -0,57 | -7,45 | 39,95 | 31,86 | Orangé | Jaune décoloré |
| Ex 3 | 92,29 | 96,65 | -0,6 | -1,6 | 37,96 | 7,13 | Orangé | Décoloré |
| Ex 4 | 92,11 | 91,58 | -1,46 | -8,76 | 40,12 | 27,12 | Orange | Jaune |
| Ex 5 | | | 0,45 | -4,43 | 36,95 | 9,37 | Orange | Jaune |
| Ex 6 (invention) | 92,27 | 97,14 | 1,14 | 1,07 | 4,11 | 4,12 | Rose pâle | Rose pâle |
| Ex 7 | | | 1,41 | -1,34 | 4,02 | 3,01 | Rose pâle | Décoloré |
| Ex 8 | 98,97 | 100,2 | 1,95 | -0,61 | 2,61 | 1,54 | Rose pâle | Décoloré |

Les exemples 1 et 6 de parfums colorés selon l'invention comprenant l'association octocrylene/butylmethoxydibenzoylmethane présentent une couleur stable après 16 heures d'exposition au simulateur solaire contrairement aux exemples 2 à 5 et 7-8.

### Exemple 9 : Parfum hydrosiliconé

| Ingredients | Quantités |
|---|---|
| Parfum du type fruité floral selon les | 15,00 |
| exemples 6 à 8 | |
| BHT | 0,0058 |
| Red 4 | 0,000091 |
| Dimethicone (DOW CORNING | 7,44 |
| FLUID 200 1,5 cst) | |
| Octocrylene | 0,58 |
| Butyl Methoxy Dibenzoylmethane | 0,58 |
| Ethanol | 71,36 |
| Eau | qsp 100 |

## Revendications

1. Composition parfumée colorée comprenant dans un milieu cosmétiquement acceptable
a) au moins 1 % en poids par rapport au poids total de la composition d'au moins une substance parfumante ;
b) au moins un alcool volatil et/ou une huile de silicone volatile ,
c) au moins un colorant soluble dans ledit milieu,
d) au moins un composé β,β'-diphénylacrylate d'alkyle ou α-cyano-β,β'-diphénylacrylate d'alkyle dans une quantité d'au moins 0,5% en poids par rapport au poids total de la composition et
e) au moins un filtre UV-A soluble dans ledit milieu.
f) et éventuellement de l'eau.

2. Composition selon la revendication 1, où la quantité de substance parfumante varie de 1 à 30 % en poids, mieux de 2 à 25% en poids par rapport au poids total de la composition.

3. Composition selon l'une quelconque des revendications 1 et 2, où le β,β'-diphénylacrylate d'alkyle ou α-cyano-β,β'-diphénylacrylates d'alkyle est choisi parmi ceux répondant à la formule (I) suivante : 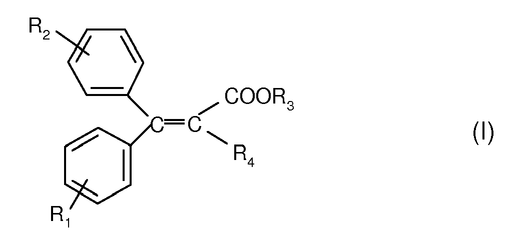 dans laquelle :
- R₁ et R₂, identiques ou différents, sont en position méta ou para et sont choisis parmi : l'hydrogène; un radical alcoxy en C₁-C₈ à chaîne droite ou ramifiée; un radical alkyle en C₁-C₄ à chaîne droite ou ramifiée;
- R₃ représente un radical alkyle en C₁-C₁₂ à chaîne droite ou ramifiée;
- R₄ représente un atome d'hydrogène ou un radical -CN.

4. Composition selon l'une quelconque des revendications 1 à 3, où le β,β'-diphénylacrylate d'alkyle ou α-cyano-β,β'-diphénylacrylates d'alkyle est choisi parmi l'α-cyano-β,β' diphénylacrylate de 2-éthylhexyle ou encore l'α-cyano-β, β'-diphénylacrylate d'éthyle.

5. Composition selon la revendication 4, où le β,β'-diphénylacrylate d'alkyle ou α-cyano-β,β'-diphénylacrylates d'alkyle est l'α-cyano-β,β' diphénylacrylate de 2-éthylhexyle.

6. Composition selon l'une quelconque des revendications 1 à 5, où le ou les filtres UV-A solubles dans le milieu de la composition sont choisis parmi les dérivés de benzophénone, les dérivés de dibenzoylméthane, les dérivés de benzylidène camphre sulfoniques, les silicones benzotriazoles.

7. Composition selon la revendication 6, où les dérivés de benzophénone sont choisis parmi :
Benzophenone-2
Benzophenone-3
2-(4-diethylamino-2-hydroxybenzoyl)-benzoate de n-hexyle

8. Composition selon la revendication 6, où le dérivé de benzylidènecamphre sulfonique est le Terephthalylidene Dicamphor Sulfonic Acid.

9. Composition selon la revendication 6, où les silicones benzotriazole sont choisies parmi les composés de formule générale (II) suivante : 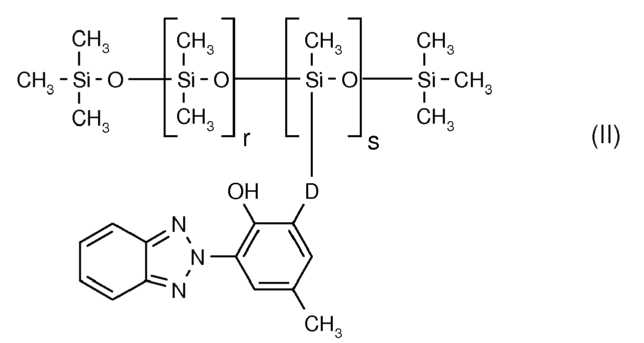 avec
0 ≤ r ≤ 15 , de préférence 0 ≤ r ≤ 10
1 ≤ s ≤ 5 , de préférence 1 ≤ s ≤ 3
et où D représente le radical divalent : 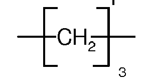 ou

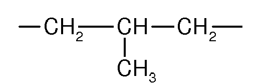

10. Composition selon la revendication 9, où la silicone benzotriazole est le Drometrizole Trisiloxane de formule (II) avec r = 0 et s = 1 et

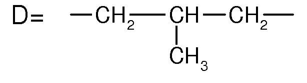

11. Composition selon la revendication 6, où les dérivés de dibenzoylméthane sont choisis parmi le 4-isopropyl-dibenzoylméthane et le 4-(ter.-butyl) 4'-méthoxy dibenzoylméthane.

12. Composition selon la revendication 11, où le filtre UVA soluble dans le milieu de la composition est le 4-(ter.-butyl) 4'-méthoxy dibenzoylméthane.

13. Composition selon l'une quelconque des revendications 1 à 12, où le ou les filtres UVA solubles sont présents à une teneur allant de 0,01% à 3% en poids, et plus particulièrement de 0,5 à 1,5% en poids par rapport au poids total de la composition.

14. Composition selon l'une quelconque des revendications 1 à 13, où le β,β'-diphénylacrylate d'alkyle ou α-cyano-β,β'-diphénylacrylates d'alkyle est présent à une teneur allant de 0,5% à 3% en poids, et plus particulièrement de 0,5 à 1,5% en poids par rapport au poids total de la composition.

15. Composition selon l'une quelconque des revendications 1 à 14, où le milieu cosmétiquement acceptable contient de l'eau dans une quantité allant de 0,01 % à 50 % et plus préférentiellement de 0,5 % à 25 % en poids par rapport au poids total de la composition.

16. Composition selon l'une quelconque des revendications 1 à 15, où l'alcool volatil est choisi parmi les monoalcools inférieurs en C₁-C₅.

17. Composition selon la revendication 16, où l'alcool volatil est l'éthanol.

18. Composition selon l'une quelconque des revendications 1 à 17, où l'alcool volatil ou les alcools volatils sont présents dans des quantités allant de 40 à 80% et de préférence dans des concentrations allant de 55 à 80% en poids par rapport au poids total de la composition.

19. Composition selon l'une quelconque des revendications 1 à 18, où les huiles siliconées volatiles sont choisies parmi les huiles de silicones linéaires ou cycliques volatiles ayant une viscosité ≤ 6 centistokes (6.10⁻⁶ m²/s), et ayant notamment de 2 à 10 atomes de silicium, ces silicones comportant éventuellement des groupes alkyles ou alkoxy ayant de 1 à 22 atomes de carbone.

20. Composition selon la revendication 19, où les huiles siliconées volatiles sont choisies parmi l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

21. Composition selon l'une quelconque des revendications 1 à 20, où les huiles siliconées volatiles sont présentes dans des quantités allant de 40 à 98,5% de préférence dans des concentrations allant de 10% à 80 % en poids par rapport au poids total de la composition.

22. Composition selon l'une quelconque des revendications 1 à 21, comprenant en plus un ou plusieurs filtres UV organiques supplémentaires actifs dans l'UV-B solubles dans le milieu de la composition.

23. Composition selon la revendication 22, où les filtres UV organiques UV-B complémentaires sont choisis parmi les dérivés cinnamiques, les dérivés salicyliques les dérivés de benzylidenecamphe alkylsulfates cationiques et leurs mélanges.

24. Composition selon la revendication 23, où les filtres UV organiques complémentaires sont choisis parmi
Ethylhexyl Methoxycinnamate
Homosalate
Ethylhexyl Salicylate
Camphor Benzalkonium Methosulfate
et leurs mélanges.

25. Composition selon l'une quelconque des revendications 22 à 24, où le ou les agents photoprotecteurs UVB supplémentaires sont présents dans des quantités allant de 0,01 à 3 % en poids et plus préférentiellement de 0,5 à1,5% en poids par rapport au poids total de la composition.

26. Composition selon l'une quelconque des revendications 1 à 25, où le ou les colorants sont présents dans des quantités allant de 10⁻⁵ à 1 % du poids total de la composition, de préférence de 10⁻⁴ à 0,1% du poids total de la composition.

27. Composition selon l'une quelconque des revendications 1 à 26, comprenant en plus au moins un additif choisi parmi les antioxydants, les actifs cosmétiques ou dermatologiques comme les émollients ou adoucissants, les agents hydratants comme la glycérine, les agents apaisants, les vitamines, les acides gras essentiels, les agents répulsifs contre les insectes, les propulseurs, les peptisants, des charges, des nacres, des paillettes et leurs mélanges.

28. Composition selon l'une quelconque des revendications 1 à 27, se présentant sous forme d'eau fraîche, d'eau de toilette, d'eau de parfum, de lotion après-rasage, d'eau de soin, d'huile de soin siliconée ou hydrosiliconée, de lotion biphasique..

29. Composition selon l'une quelconque des revendications 1 à 28, **caractérisée par le fait qu'**elle se présente sous forme de lotion conditionnée sous forme de flacon, sous forme de pompe non-aérosol ou de récipient aérosol.

30. Utilisation d'au moins un composé β,β'-diphénylacrylate d'alkyle ou α-cyano-β,β'-diphénylacrylate d'alkyle dans une concentration d'au moins 0,5% en poids par rapport au poids de la composition et d'au moins un filtre organique UV-A soluble tels que définis dans les revendications précédentes dans une composition parfumée colorée comprenant dans un milieu cosmétiquement acceptable
a) au moins 1% en poids par rapport au poids total de la composition d'au moins une substance parfumante ;
b) au moins un alcool volatil et/ou une huile de silicone volatile
c) au moins un colorant soluble dans ledit milieu
d) et éventuellement de l'eau, comme agent stabilisant de la couleur de ladite composition.

31. Procédé cosmétique de parfumage des matières kératiniques humaines et notamment de la peau, des lèvres et des phanères, comprenant l'application sur les matières kératiniques de la composition telle que définie dans les revendications 1 à 29.
